# EUROPEAN PATENT SPECIFICATION

(11) **EP 3 395 971 B1**
(45) Date of publication and mention of the grant of the patent: **25.08.2021**
(21) Application number: 16877669.8
(22) Date of filing: 16.12.2016
(51) Int. Cl.: C22C 18/00, A61L 31/02

(54) **ALLOY MATERIAL AND APPLICATION THEREOF**
LEGIERUNGSMATERIAL UND ANWENDUNG DAVON
MATÉRIAU D'ALLIAGE ET SON APPLICATION

(30) Priority: 23.12.2015 CN 201510981436
(43) Date of publication of application: 31.10.2018
(73) Proprietor: Shanghai MicroPort Medical (Group) Co., Ltd., Shanghai 201203 (CN)
(72) Inventor: CAO, Yating, Shanghai 201203 (CN); YAO, Yao, Shanghai 201203 (CN); LI, Junfei, Shanghai 201203 (CN); LUO, Qiyi, Shanghai 201203 (CN)
(74) Representative: Patentship Patentanwaltsgesellschaft mbH
(86) International application number: PCT/CN2016/110281
(87) International publication number: WO 2017/107858

(56) References cited:
- CN-A- 103 290 264
- CN-A- 104 195 368
- CN-A- 104 195 368
- CN-A- 104 212 998
- CN-A- 104 212 998
- CN-A- 105 063 427
- CN-A- 105 063 427
- JP-A- S62 112 766

## Description

### TECHNICAL FIELD

The invention relates to the field of medical technology and, in particular, to an alloy material usable in implantable medical devices and application thereof.

### BACKGROUND

With the development of medicine and science, some temporary implants such as sutures, bone fracture fixation plates and vascular stents are desired for temporary support, fixation and replacement of some biological tissues and gradual degradation and absorption with the regeneration of the tissues or organs, with a minimized long-term impact on the body.

Vascular stents are implantable mesh devices for the treatment of cardiovascular diseases such as coronary artery disease. Coronary stents can effectively avoid the medical issues arising from simple balloon dilatation and have found extensive use in coronary interventional treatment. Degradable stents are considered as the "fourth technological innovation" subsequent to PTCA, BMS and DES, which can be gradually degraded and absorbed after dilating blood vessels, thereby restoring the structure and functionality of the blood vessels.

Current research of degradable stents focuses mainly on two aspects which are degradable polymer stents and degradable metal stents. The currently researched degradable metal stents are mainly made of iron alloys, magnesium alloys and zinc alloys. For example, Patent of Application Publication No. CN102228721A describes an iron-based alloy material with a macromolecular degradable coating. CN104212998 discloses a Zn-Mg alloy comprising microelement such as Sr. CN105063427 discloses a magnetic compatible zinc alloy for MRI use. CN104195368 discloses a Zn-Sr alloy comprising at least one microelement such as Si, P, Li, Ag, Sn. However, the existing iron alloy stents degrade too slowly to reduce undesirable reconstruction of the vascular system, and magnesium alloy stents degrade too fast to provide insufficient support for blood vessels. There are few reports about zinc alloys, and the stents made of pure zinc suffer from a series of problems such as insufficient strengths and uncontrollable degradation rates.

Therefore, there is an urgent need in the art for a degradable material that addresses the issues such as low strength, inferior plasticity and mismatch between degradation rate and body requirements and is suited to use in implantable medical devices such as degradable metal stents.

### SUMMARY OF THE INVENTION

It is an objective of the present invention to provide a degradable material with high mechanical performance, a desirable degradation rate and good biocompatibility as well as an implantable medical device possessing these properties.

In the present invention, the alloy material of claim 1 is provided.

In a preferred embodiment, the magnesium is present in a percentage of 0.01%-2.0%, more preferably 0.1%-1.0%.

In another preferred embodiment, the selenium is present in a percentage of 0.09%-0.2%.

In another preferred embodiment, the strontium is present in a percentage of 0.05%-0.3%, more preferably 0.09%-0.2%.

In an aspect of the present invention, an implantable medical device using the alloy material as defined above is provided.

In another preferred embodiment, the implantable medical device is a medical intraluminal stent or an orthopedic implant. The medical intraluminal stent may be but not limited to be a coronary stent, an aortic stent, an intracranial stent, a peripheral stent, an intraoperative stent, a valvular stent, a biliary stent, an esophageal stent, an intestinal stent, a pancreatic stent, a urethral stent or a tracheal stent. The orthopedic implant may be but not limited to be a bone nail, a bone screw or a bone plate.

On such a basis, the present invention provides a degradable material that addresses the issues such as low strength, inferior plasticity and mismatch between degradation rate and body requirements and is suited to use in implantable medical devices such as degradable metal stents.

### BRIEF DESCRIPTION OF THE DRAWINGS

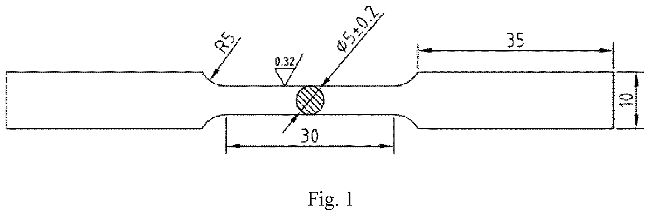

Fig. 1 shows the dimensions of a specimen provided for a room-temperature tensile test in Example 3.

### DETAILED DESCRIPTION

After extensive and in-depth research, the inventors have found that the degradation rate of a zinc alloy can be made controllable and mechanical properties thereof can be improved by adding thereto, as alloy elements, suitable amounts of magnesium (Mg), selenium (Se) and strontium (Sr) and this invention was attained based on this finding.

### Alloy Material

An alloy material according to this invention consists of zinc (Zn), magnesium (Mg), selenium (Se) and strontium (Sr). With respect to the total weight of the alloy material, zinc is present in a weight percentage of 97-99%, magnesium in a weight percentage of less than 3% and greater than 0, selenium in a weight percentage of 0.05-0.3% and strontium in a weight percentage of 0.001-0.5%. Except for the magnesium, selenium and tellurium, the remainder of the alloy material is consisted of zinc.

All the metals used in the fabrication of the alloy material of the present invention have a purity of greater than or equal to 99.99%. In other words, the zinc has a purity of greater than or equal to 99.99%; the magnesium has a purity of greater than or equal to 99.99%; the selenium has a purity of greater than or equal to 99.99%; and the strontium has a purity of greater than or equal to 99.99%.

In one embodiment, the magnesium is present at a percentage by weight of 0.01-2.0%, with 0.1-1.0% being preferred. An increase in the magnesium content may degrade mechanical properties of the alloy material.

The selenium is present at a percentage by weight of 0.05-0.3%, preferably 0.09-0.2%.

In one embodiment, the strontium is present at a percentage by weight of 0.05-0.3%, preferably 0.09-0.2%.

The alloy material of the present invention may be fabricated using any suitable conventional method in art, for example, but not limited to, by mixing zinc, magnesium, strontium and selenium in the percentages described above, smelting the mixture under the protection of CO₂ and SF₆ gases, rapidly cooling the smelted mixture into a zinc alloy ingot, and subjecting the zinc alloy ingot to peeling, homogenized heat treatment and rolling process.

The alloy material may be fabricated into a variety of shapes including bars, chunks, balls and rollers, depending on the application where it is used.

### Implantable Medical Device

As used herein, an "implantable medical device" refers to any instrument that is partially or wholly introduced into the body or a natural lumen via a surgical or interventional procedure and remains partially or wholly within the body for a long term of at least 30 days after the introduction.

An implantable medical device according to the present invention employs the alloy material as defined above. In a preferred embodiment of the present invention, the implantable medical device is a medical intraluminal stent or an orthopedic implant.

As used herein, a "stent" refers to a tubular device implantable into a lumen of the human body via an interventional procedure. A stent according to the present invention is made of the alloy material as defined above. In a preferred embodiment of the present invention, the stent is provided with a conformation for releasing a drug, for example, a coating or a reservoir.

The stent according to the present invention may include coronary stents, aortic stents, intracranial stents, peripheral stents, intraoperative stents, valvular stents, biliary stents, esophageal stents, intestinal stents, pancreatic stents, urethral stents and tracheal stents. The orthopedic implant according to the present invention may include bone nails, bone screws, and bone plates.

Unless defined otherwise herein, all technical and scientific terms used herein have the same meaning as commonly understood by one of ordinary skill in the art to which this invention pertains. In addition, unless the context indicates otherwise, singular forms of the terms herein are to be construed as including the plural form and vice versa.

The present invention essentially offers the following advantages:
1. Degradation products of the elements contained in the alloy material according to the present invention can contribute to the regulation of various functions of the human body and can be completely metabolically degraded.
2. The alloy material used in the implantable medical device according to the present invention is degraded within a period of more than six months and can thus provide mechanical support for a sufficiently long time. In case of the implantable medical device being an intraluminal stent, it can prevent the occurrence of secondary stenosis.
3. The combination of magnesium, selenium and strontium with zinc as a matrix imparts good mechanical properties to the alloy material used in the implantable medical device according to the present invention.

The present invention will be described in greater detail below with reference to a few specific Examples. It is to be understood that these Examples are presented only for illustrating the invention rather than limiting the scope thereof. In the Examples, any test with its conditions not being specified was generally performed under conventional conditions or manufacturer' recommended conditions. Additionally, all percentages, ratios, proportions or parts are given herein by weight, unless otherwise indicated.

In this specification, percentages by weight are expressed in units of measurement well known to those skilled in the art, for example, those representing the percentages of the weights of constituent elements of a compound to the total weight thereof.

Unless defined otherwise herein, all technical and scientific terms used herein have the same meaning as commonly understood by one of ordinary skill in the art to which this invention pertains. Preferred methods and materials are now described by way of examples.

In the Examples, the zinc had a purity of greater than or equal to 99.99%, the magnesium had a purity of greater than or equal to 99.99%, the strontium had a purity of greater than or equal to 99.99%, the selenium had a purity of greater than or equal to 99.99%, and hence a content of the impurity was ≤ 0.01%. The materials in the following examples were purchased from Shanghai Benqili Hardware Co., Ltd.

### Example 1

### Alloy Material 1

Alloy Material 1 was prepared with the following composition shown in Table 1.

**Table 1 Composition of Alloy Material 1**

| Element | Mg | Sr | Se | Zn |
|---|---|---|---|---|
| Weight Percentage | 0.15% | 0.1% | 0.1% | Remainder |

The metals listed in the above table, i.e., zinc, magnesium, strontium and selenium, were mixed so that they were present in the resulting mixture in the respective weight percentages as shown, melted at 500 °C under the protection of CO₂ and SF₆ gases and cooled with circulating water to result in a zinc alloy ingot which then sequentially underwent a peeling process, a homogenized heat treatment performed at 200 °C for 30 minutes and a rolling process in which the zinc alloy ingot was hot-rolled at a temperature controlled at about 250 °C in several passes at a rolling rate controlled at 10-20% per pass so that about 70% of it was totally rolled, resulting in Alloy Material 1 having the composition shown in Table 1.

### Example 2

### Alloy Material 2

Alloy Material 2 was prepared with the following composition shown in Table 2.

**Table 2 Composition of Alloy Material 2**

| Element | Mg | Sr | Se | Zn |
|---|---|---|---|---|
| Weight Percentage | 1% | 0.15% | 0.1% | Remainder |

The metals listed in the above table, i.e., zinc, magnesium, strontium and selenium, were mixed so that they were present in the resulting mixture in the respective weight percentages as shown, melted at 550 °C under the protection of CO₂ and SF₆ gases and cooled with circulating water to result in a zinc alloy ingot which then sequentially underwent a peeling process, a homogenized heat treatment performed at 200 °C for 45 minutes and a rolling process in which the zinc alloy ingot was hot-rolled at a temperature controlled at about 250 °C in several passes at a rolling rate controlled at 10-20% per pass so that about 70% of it was totally rolled, resulting in Alloy Material 2 having the composition shown in Table 2.

Differing from Example 1, the Sr content of Example 2 was 0.15% that was greater than the Se content. On the one hand, Sr could refine the texture of the material and thus impart higher performance (grain refining strengthening) thereto. On the other hand, Sr had a slightly higher burn-out rate than Se, so it was reasonable that the Sr content was higher than the Se content.

### Example 3

### Mechanical Properties of Alloy Materials

Ambient-temperature tensile tests were performed on specimens with the dimensions shown in Fig. 1 obtained by lathing ϕ 10 mm × 110 mm round bars of Alloy Materials 1 and 2 prepared in Examples 1 and 2 pursuant to GB/T 228-2002 - Metallic Materials - Tensile Testing at Ambient Temperature - to determine their mechanical properties including the yield strengths, tensile strengths and elongations at break. Three parallel specimens were tested for each of the materials and their measurements results were averaged to determine the yield strengths, tensile strengths and elongations at break of the materials.

Mechanical properties of the rolled specimens (i.e., the specimens fabricated from the rolled alloy materials of Examples 1 and 2) were summarized in Table 3.

**Table 3 Test Results of Mechanical Properties of Alloy Material**

| Specimen No. | Tensile Strength (MPa) | Yield Strength (MPa) | Elongation at Break (%) |
|---|---|---|---|
| Alloy Material 1 | 270 | 210 | 12 |
| Alloy Material 2 | 350 | 200 | 4 |
| WE43 Magnesium Alloy (purchased from Dongguan Yiwan metal materials Co., Ltd.) | 300 | 250 | 6 |

### Example 4

### Degradation Rates of Alloy Materials

A Hank's solution was prepared by sequentially dissolving 8.0 g of NaCl, 0.35 g of NaHCO₃, 0.4 g of KCl, 0.1 g of glucose, 0.06 g of K₂HPO₄ and 0.06 g of NaH₂PO₄ in water, adjusting the pH of the solution to 7.2-7.4 with NaHCO₃, pouring the solution into a measuring flask and increasing the volume of the solution to 1,000 ml.

After immersed in the Hank's solution, Alloy Materials 1 and 2 obtained in Examples 1 and 2 were observed to each have a degradation rate much lower than that of the WE43 magnesium alloy, i.e., 0.34 mm per year. Alloy Materials 1 and 2 obtained in the Examples could be used to fabricate zinc alloy stents capable of providing defective blood vessels with radial support for over 6 months as well as bone plates capable of providing fixation and mechanical support for at least half a year.

The foregoing description presents merely a few preferred embodiments of the present invention and is not intended to limit the true scope thereof that is broadly defined by the appended claims.

## Claims

1. An alloy material, comprising the following elements in weight percentages given:
magnesium: less than 3%;
selenium:0.05%-0.3%;
strontium: 0.001%-0.5%;
zinc: the remainder.

2. The alloy material according to claim 1, wherein the magnesium is present in a percentage of 0.01 %-2.0%.

3. The alloy material according to claim 1, wherein the magnesium is present in a percentage of 0.1%-1.0%.

4. The alloy material according to claim 1, wherein the selenium is present in a percentage of 0.09%-0.2%.

5. The alloy material according to claim 1, wherein the strontium is present in a percentage of 0.05%-0.3%.

6. The alloy material according to claim 5, wherein the strontium is present in a percentage of 0.09%-0.2%.

7. An implantable medical device using the alloy material as defined in any one of claims 1 to 6.

8. The implantable medical device according to claim 7, which is a medical intraluminal stent or an orthopedic implant.

9. The implantable medical device according to claim 8, wherein the medical intraluminal stent is a coronary stent, an aortic stent, an intracranial stent, a peripheral stent, an intraoperative stent, a valvular stent, a biliary stent, an esophageal stent, an intestinal stent, a pancreatic stent, a urethral stent or a tracheal stent.

10. The implantable medical device according to claim 8, wherein the orthopedic implant is a bone nail, a bone screw or a bone plate.

## Patentansprüche

1. Legierungsmaterial, umfassend folgende, in Gewichtsprozenten angegebene, Elemente:
Magnesium: weniger als 3%;
Selen: 0,05%-0,3%;
Strontium: 0,001%-0,5%;
Zink: der Restbestand.

2. Legierungsmaterial nach Anspruch 1, wobei das Magnesium in einem Prozentsatz von 0,01%-2,0% vorhanden ist.

3. Legierungsmaterial nach Anspruch 1, wobei das Magnesium in einem Prozentsatz von 0,1%-1,0% vorhanden ist.

4. Legierungsmaterial nach Anspruch 1, wobei das Selen in einem Prozentsatz von 0,09%-0,2% vorhanden ist.

5. Legierungsmaterial nach Anspruch 1, wobei das Strontium in einem Prozentsatz von 0,05%-0,3% vorhanden ist.

6. Legierungsmaterial nach Anspruch 5, wobei das Strontium in einem Prozentsatz von 0,09%-0,2% vorhanden ist.

7. Implantierbare medizinische Vorrichtung, welche das Legierungsmaterials nach einem der Ansprüche 1 bis 6 verwendet.

8. Implantierbare medizinische Vorrichtung nach Anspruch 7, welche ein medizinischer intraluminaler Stent oder ein orthopädisches Implantat ist.

9. Implantierbare medizinische Vorrichtung nach Anspruch 8, wobei der medizinische intraluminale Stent ein koronarer Stent, ein Aortenstent, ein intrakranieller Stent, ein peripherer Stent, ein intraoperativer Stent, ein Klappenstent, ein Gallenstent, ein Ösophagusstent, ein Darmstent, ein Pankreasstent, ein Harnröhrenstent oder ein Trachealstent ist.

10. Implantierbare medizinische Vorrichtung nach Anspruch 8, wobei das orthopädische Implantat ein Knochennagel, eine Knochenschraube oder eine Knochenplatte ist.

## Revendications

1. Matériau d'alliage, comprenant les éléments suivants dans les pourcentage en poids indiqués :
magnésium : moins de 3 % ;
sélénium : 0,05 % à 0,3 % ;
strontium : 0,001 % à 0,5 % ;
zinc : le reste.

2. Matériau d'alliage selon la revendication 1, dans lequel le magnésium est présent dans un pourcentage de 0,01 % à 2,0 %.

3. Matériau d'alliage selon la revendication 1, dans lequel le magnésium est présent dans un pourcentage de 0,1 % à 1,0 %.

4. Matériau d'alliage selon la revendication 1, dans lequel le sélénium est présent dans un pourcentage de 0,09 % à 0,2 %.

5. Matériau d'alliage selon la revendication 1, dans lequel le strontium est présent dans un pourcentage de 0,05 % à 0,3 %.

6. Matériau d'alliage selon la revendication 5, dans lequel le strontium est présent dans un pourcentage de 0,09 % à 0,2 %.

7. Dispositif médical implantable utilisant le matériau d'alliage tel que défini dans l'une quelconque des revendications 1 à 6.

8. Dispositif médical implantable selon la revendication 7, qui est un stent médical intraluminal ou un implant orthopédique.

9. Dispositif médical implantable selon la revendication 8, où le stent médical intraluminal est un stent coronarien, un stent aortique, un stent intracrânien, un stent périphérique, un stent peropératoire, un stent valvulaire, un stent biliaire, un stent œsophagien, un stent intestinal, un stent pancréatique, un stent urétral ou un stent trachéal.

10. Dispositif médical implantable selon la revendication 8, où l'implant orthopédique est un clou osseux, une vis osseuse ou une plaque osseuse.
